# EUROPEAN PATENT APPLICATION

(11) **EP 4 501 206 A1**
(43) Date of publication of application: **05.02.2025**
(21) Application number: 22933492.5
(22) Date of filing: 25.03.2022
(51) Int. Cl.: A61B 1/045

(54) **IMAGE PROCESSING DEVICE, IMAGE PROCESSING METHOD, AND STORAGE MEDIUM**

(71) Applicant: NEC Corporation, 108-8001 Tokyo (JP)
(72) Inventor: IWADATE, Yuji, Tokyo 108-8001 (JP); MAEDA, Naoto, Tokyo 108-8001 (JP)
(74) Representative: Betten & Resch
(86) International application number: PCT/JP2022/014404
(87) International publication number: WO 2023/181353

(57) **Abstract**

The image processing device 1X includes an acquisition means 30X, a determination means 31X, and a detection means 33X. The acquisition means 30X is configured to acquire an endoscopic image obtained by photographing an examination target by a photographing unit provided in an endoscope. The determination means 31X is configured to determine, based on the endoscopic image, a state of the examination target or a photographed part of the examination target. The detection means 33X is configured to detect, based on the state or the photographed part and the endoscopic image, a part of interest to be noticed in the examination target.

## Description

### TECHNICAL FIELD

The present disclosure relates to a technical field of an image processing device, an image processing method, and a storage medium for processing an image to be acquired in endoscopic examination.

### BACKGROUND

An endoscopic examination system for displaying images taken in the lumen of an organ is known. For example, Patent Literature 1 discloses an endoscopic examination system that detects a region of interest based on an endoscopic image and a target region detection threshold value and that determines whether the region of interest is either a flat lesion or a raised lesion. Further, Patent Literature 2 discloses an endoscopic examination system configured to perform a three-dimensional reconstruction of a target organ of examination by the SfM (Structure from Motion) using time-series endoscopic images and identify a captured region through matching (image registration) between the three-dimensionally reconstructed data and a standard model of the target organ thereby to display the identified captured region.

### CITATION LIST

### PATENT LITERATURE

Patent Literature 1: WO2019/146077
Patent Literature 1: WO2021/234907

### SUMMARY

### PROBLEM TO BE SOLVED

In the case of detecting a part (region) of interest such as a lesion part from the image taken in the endoscopic examination, since the degree of difficulty in detecting the lesion part differs depending on the photographed portion and/or the presence or absence of inflammation, it is necessary to carry out the lesion part detection according to the situation.

In view of the above-described issue, it is therefore an example object of the present disclosure to provide an image processing device, an image processing method, and a storage medium capable of accurately detecting a lesion in an endoscope examination.

### MEANS FOR SOLVING THE PROBLEM

One mode of the image processing device is an image processing device including:
an acquisition means configured to acquire an endoscopic image obtained by photographing an examination target by a photographing unit provided in an endoscope;
a determination means configured to determine, based on the endoscopic image, a state of the examination target or a photographed part of the examination target; and
a detection means configured to detect, based on the state or the photographed part and the endoscopic image, a part of interest to be noticed in the examination target.

One mode of the image processing method is an image processing method executed by a computer, the image processing method including:
acquiring an endoscopic image obtained by photographing an examination target by a photographing unit provided in an endoscope;
determining, based on the endoscopic image, a state of the examination target or a photographed part of the examination target; and
detecting, based on the state or the photographed part and the endoscopic image, a part of interest to be noticed in the examination target.

One mode of the storage medium is a storage medium storing a program executed by a computer, the program causing the computer to:
acquire an endoscopic image obtained by photographing an examination target by a photographing unit provided in an endoscope;
determine, based on the endoscopic image, a state of the examination target or a photographed part of the examination target; and
detect, based on the state or the photographed part and the endoscopic image, a part of interest to be noticed in the examination target.

### EFFECT

An example advantage according to the present invention is to accurately detect a lesion in accordance with a condition of the examination target or a photographed portion of the examination target.

### BRIEF DESCRIPTION OF THE DRAWINGS

[FIG. 1] It illustrates a schematic configuration of an endoscopic examination system.
[FIG. 2] It illustrates a hardware configuration of an image processing device.
[FIG. 3] It is a functional block diagram of an image processing device related to the lesion detection process.
[FIG. 4] It is a functional block diagram of a lesion detection unit.
[FIG. 5] It illustrates the first display example of the display screen image displayed on a display device in the endoscopic examination.
[FIG. 6] It is an example of a flowchart showing an outline of a process performed by the image processing device during the endoscopic examination in the first example embodiment.
[FIG. 7] It is an example of a functional block diagram of the image processing device according to a first modification.
[FIG. 8] It illustrates a second display example of the display screen image displayed on the display device in the endoscopic examination in the first modification.
[FIG. 9] It is an example of a functional block diagram of the image processing device in the first example in the second modification.
[FIG. 10] It is an example of a functional block diagram of the image processing device in the second example in the second modification.
[FIG. 11] It is a schematic configuration diagram of an endoscopic examination system according to the fourth modification.
[FIG. 12] It is a block diagram of an image processing device according to the second example embodiment.
[FIG. 13] It is an example of a flowchart executed by the image processing device in the second example embodiment.

### EXAMPLE EMBODIMENTS

Hereinafter, example embodiments of an image processing device, an image processing method, and a storage medium will be described with reference to the drawings.

### <First Example Embodiment>

### (1) System Configuration

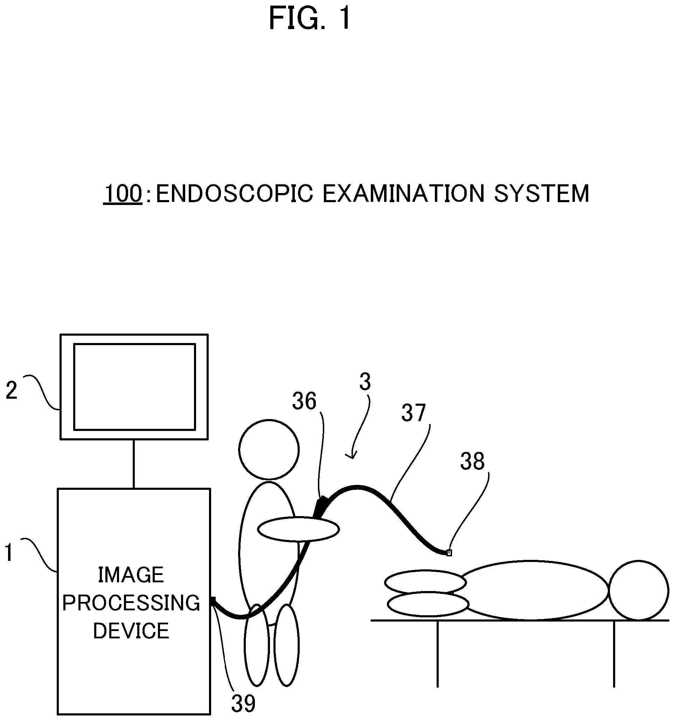

FIG. 1 shows a schematic configuration of an endoscopic examination system 100. As shown in FIG. 1, an endoscopic examination system 100 is a system configured to inform an examiner, such as a doctor who performs examination or treatment using an endoscope, of a part (also referred to as "lesion part") of examination target which is suspected of a lesion, and mainly includes an image processing device 1, a display device 2, and an endoscope 3 connected to the image processing device 1. The lesion part is an example of the "part of interest".

The image processing device 1 acquires an image (also referred to as "endoscopic image Ia") captured by the endoscope 3 in time series from the endoscope 3 and displays a screen image based on the endoscopic image Ia on the display device 2. The endoscopic image Ia is an image captured at predetermined time intervals in at least one of the insertion process of the endoscope 3 to the subject and/or the ejection process of the endoscope 3 from the subject. In the present example embodiment, the image processing device 1 analyzes the endoscopic image Ia to detect the lesion part in the endoscopic image Ia, and displays the information on the detection result on the display device 2.

The display device 2 is a display or the like for display information based on the display signal supplied from the image processing device 1.

The endoscope 3 mainly includes an operation unit 36 for examiner to perform a predetermined input, a shaft 37 which has flexibility and which is inserted into the organ to be photographed of the subject, a tip unit 38 having a built-in photographing unit such as an ultrasmall image pickup device, and a connecting unit 39 for connecting to the image processing device 1.

The configuration of the endoscopic examination system 100 shown in FIG.1 is an example, and various change may be applied thereto. For example, the image processing device 1 may be configured integrally with the display device 2. In another example, the image processing device 1 may be configured by a plurality of devices.

It is noted that the target of the endoscopic examination in the present disclosure is not limited to a large bowel, it may be any organ subject to endoscopic examination such as esophagus, stomach, pancreas. Examples of the target of the endoscopic examination in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope. In addition, the conditions (referred to as "target conditions") of the lesion part to be detected in endoscopic examination are exemplified as (a) to (f) below.
(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids.

### (2) Hardware Configuration

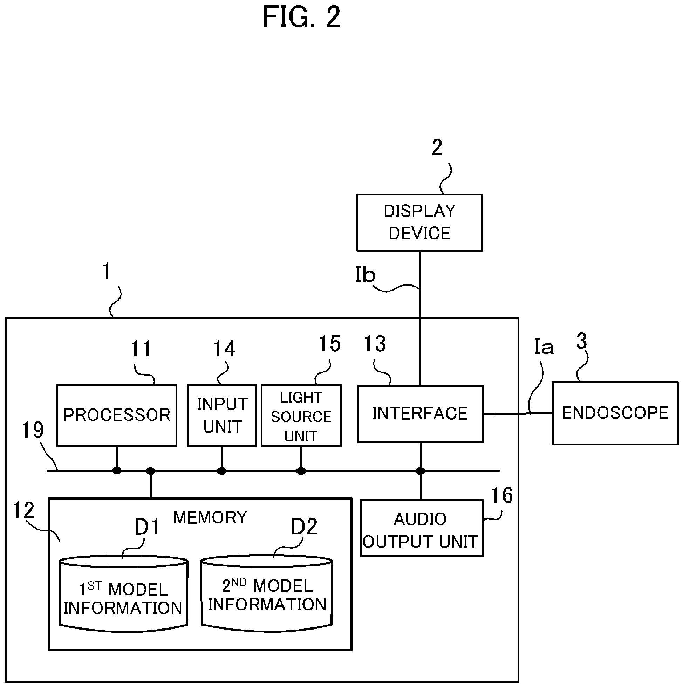

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 is an example of a computer. The processor 11 may be configured by a plurality of processors. The processor 11 is an example of a computer.

The memory 12 is configured by a variety of volatile memories which is used as working memories, and nonvolatile memories which stores information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each process in the present example embodiment.

The memory 12 stores first model information D1 relating to a first model used to make a determination as to the condition (also referred to as "background condition") other than the target condition to be detected in the endoscopic examination, and second model information D2 relating to a second model used to detect the target condition. Examples of the background condition include inflammation, bleeding, Barrett's esophagus if the examination target is an esophagus, and any other conditions other than the target condition. The first model information D1 and the second model information D2 will be described later.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides an electrical signal to the processor 11 indicative of the endoscopic image Ia supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the tip unit 38 of the endoscope 3. The light source unit 15 may also incorporate a pump or the like for delivering water and air to be supplied to the endoscope 3. The audio output unit 16 outputs a sound under the control of the processor 11.

Next, the first model information D1 and the second model information D2 stored in the memory 12 will be described in detail.

The first model information D1 is information relating to the first model configured to output information relating to the background condition when an endoscopic image is inputted to the first model. The first model data D1 contains the parameters required to build the first model. For example, the first model is a classification model configured to output a classification result regarding the presence or absence (and the degree) of the background condition when an endoscopic image is inputted to the model. The classification result outputted by the first model may indicate the presence or absence (and the degree) of a particular type of the background condition, or may indicate the detected type (and the degree) of the background condition. The first model may be any machine learning model (including any statistical model, hereinafter the same) such as a neural network and a support vector machine. Examples of the typical model of such a neural network include Fully Convolutional Network, SegNet, U-Net, V-Net, Feature Pyramid Network, Mask R-CNN, and DeepLab. When the first model is constituted by a neural network, the first model information D1 includes various parameters such as a layer structure, a neuron structure of each layer, the number of filters and the size of filters in each layer, and the weight for each element of each filter.

The first model is not limited to being a machine-learning model, but may be a model configured to determine whether or not there is a particular background condition based on the RGB ratio of the inputted endoscopic image. For example, the first model may be a model configured to determine that there is a background condition (e.g., irritation), upon determining that the ratio (e.g., the average ratio of all pixels) of R among the RGB of the inputted endoscopic image is equal to or greater than a predetermined threshold value. In this instance, the above-described calculation formula for calculating the ratio of R and threshold value are previously stored in the memory 12 as the first model information D1.

The second model information D2 is information relating to the second model and includes parameters required for building the second model, wherein the second model is configured to output information regarding the target condition to be detected in the endoscopic examination when an endoscopic image is inputted to the second model. For example, the second model may be any machine learning model (including any statistical model, hereinafter the same) such as a neural network and a support vector machine. For example, when the second model is constituted by a neural network, the second model information D2 includes various parameters such as a layer structure, a neuron structure of each layer, the number of filters and filter sizes in each layer, and a weight for each element of each filter.

In the present embodiment, the second model is trained to output, when an endoscopic image is inputted thereto, at least a score (also referred to as "lesion confidence score S") indicating the confidence level of the presence of a lesion part subjected to the target condition in the inputted endoscopic image. It is herein assumed that the value of the lesion confidence score S increases with an increase in the confidence level that there is a lesion part subjected to the target condition. For example, the second model may output, as the lesion confidence score S, the confidence level outputted when a neural network is adopted as the architecture of the classification model. In some embodiments, the second model may be a model configured to output not only the lesion confidence score S but also information (also referred to as "lesion region information") indicating the position or region (area) of the lesion part in the inputted endoscopic image. In some embodiments, the second model may be a model configured to output a heat map indicating the lesion confidence score S for each unit region (e.g., a region of one or more pixels) in the inputted endoscopic image. In this case, based on the lesion confidence score S for each unit region and a threshold value to be described later, the image processing device 1 may make a determination as to whether or not the each unit region corresponds to a lesion part subjected to the target condition. The second model corresponds to a model which has learned the relation between an endoscopic image inputted to the second model and the presence or absence of a lesion part in the inputted endoscopic image.

In the case where there are multiple supposed background conditions, the first model may be provided for each background condition. In this situation, the parameters for building the first model for each background condition are included in the first model data D1. Similarly, when there are multiple supposed target conditions, the second model may be provided for each target condition. In this situation, the parameters for building the second model for each target condition are included in the second model data D2.

If the first model and the second model are learning models, the first model and the second model are trained in advance based on sets of an input image, which conforms to the input format of each model, and the corresponding correct answer data indicating a correct answer to be outputted when the input image is inputted to each model. The parameters of the models obtained by training are stored as the first model information D1 and the second model information D2 in the memory 12, respectively.

### (4) Lesion Detection Process

A description will be given of the lesion detection process which is a process related to detection of the lesion part. In summary, on the basis of the background condition determined by the first model, the image processing device 1 sets a threshold value (also referred to as "lesion threshold value") for comparing with the lesion confidence score S to determine the presence or absence of the lesion part of the target condition. As a result, the image processing device 1 accurately sets the lesion threshold value and improves the lesion detection accuracy.

### (4-1) Functional Blocks

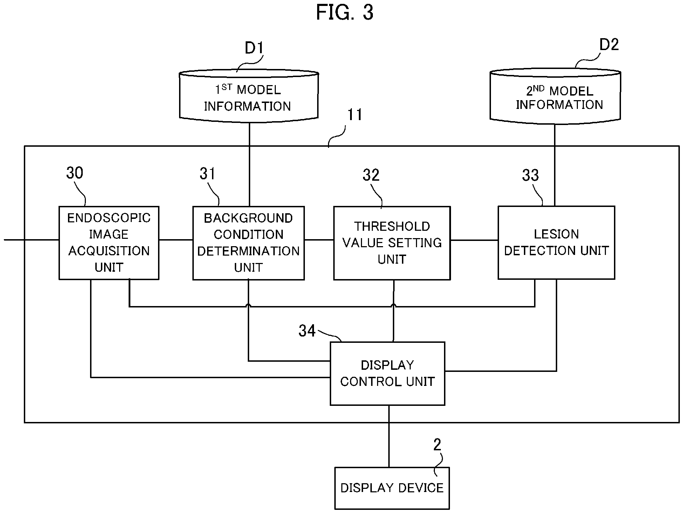

FIG. 3 is a functional block diagram of the image processing device 1 related to the lesion detection process. The processor 11 of the image processing device 1 functionally includes an endoscopic image acquisition unit 30, a background condition determination unit 31, a threshold value setting unit 32, a lesion detection unit 33, and a display control unit 34. In FIG. 3, any blocks to exchange data with each other are connected by a solid line, but the combination of the blocks to exchange data with each other is not limited thereto. The same applies to the drawings of other functional blocks described below.

The endoscopic image acquisition unit 30 acquires the endoscopic image Ia taken by the endoscope 3 through the interface 13 at predetermined intervals. Then, the endoscopic image acquisition unit 30 supplies the acquired endoscopic image Ia to the background condition determination unit 31, the lesion detection unit 33, and the display control unit 34, respectively.

Based on the endoscopic image Ia supplied from the endoscopic image acquisition unit 30, the background condition determination unit 31 makes a determination regarding the background condition and supplies information (also referred to as "background condition information") indicating the determination result of the background condition to the threshold value setting unit 32. In this case, the background condition determination unit 31 inputs the endoscopic image Ia supplied from the endoscopic image acquisition unit 30 to the first model configured by referring to the first model information D1, and makes the determination regarding the background condition based on the information outputted by the first model in response to the input of the endoscopic image Ia to the first model. For example, the first model outputs a classification result regarding the presence or absence of each of various background conditions assumed in the target organ of examination, and the background condition determination unit 31 generates background condition information indicating the type of the detected background condition, based on the classification result. In some embodiments, the classification result and background condition information described above may include information regarding the degree of the background condition when a background condition is detected. The background condition determination unit 31 may supply the background condition information to the display control unit 34.

The threshold value setting unit 32 sets the lesion threshold value based on the background condition information supplied from the background condition determination unit 31, and supplies the set lesion threshold value to the lesion detection unit 33. In this case, for example, the memory 12 stores relation information (e.g., look-up table or formula) which indicates a relation between the type of background condition and the lesion threshold value to be set, and the threshold value setting unit 32 sets, by referring to the relation information, the lesion threshold value corresponding to the background condition indicated by the background condition information. In this case, for example, the lesion detection tests based on the second model and the lesion threshold value by use of test data are conducted beforehand and then the above-mentioned relation information is generated such that the F value (harmonic mean of the precision ratio and the recall ratio) is maximized for each type of the background condition in the tests. The training data in this case is, for example, sets of an endoscopic image prepared for each type of background condition and the corresponding correct answer data indicating the presence or absence of a lesion part in the endoscopic image. The above-described relation information may indicate the relation between the type and degree of the background condition and the lesion threshold value to be set. In some embodiments, the threshold value setting unit 32 may supply the display control unit 34 with the set lesion threshold value.

On the basis of the endoscopic image Ia supplied from the endoscopic image acquisition unit 30 and the lesion threshold value supplied from the threshold value setting unit 32, the lesion detection unit 33 detects a lesion part, and supplies information (also referred to as "lesion detection information") indicating the detection result to the display control unit 34. In this case, the lesion detection unit 33 generates the lesion detection information by comparing the lesion confidence score S, which is outputted from the second model when the endoscopic image Ia is inputted into the second model configured by referring to the second model information D2, with the lesion threshold value. If the second model further outputs the lesion region information indicating the region of the lesion part in the endoscopic image Ia, the lesion detection unit 33 may include the lesion region information in the lesion detection information.

On the basis of the latest endoscopic image Ia supplied from the endoscopic image acquisition unit 30 and the lesion detection information supplied from the lesion detection unit 33, the display control unit 34 generates display information Ib and supplies the display device 2 with the generated display information Ib to thereby cause the display device 2 to display the latest endoscopic image Ia and the lesion detection result. In some embodiments, the display control unit 34 may further display information on the background condition information supplied from the background condition determination unit 31 or/and the lesion threshold value supplied from the threshold value setting unit 32 on the display device 2. The display example of the display device 2 by the display control unit 34 will be described later. In some embodiments, if the lesion detection information indicates that the lesion part has been detected, the display control unit 34 may perform the audio output control of the sound output unit 16 to output a warning sound or voice guidance or the like for notifying the user that the lesion part is detected.

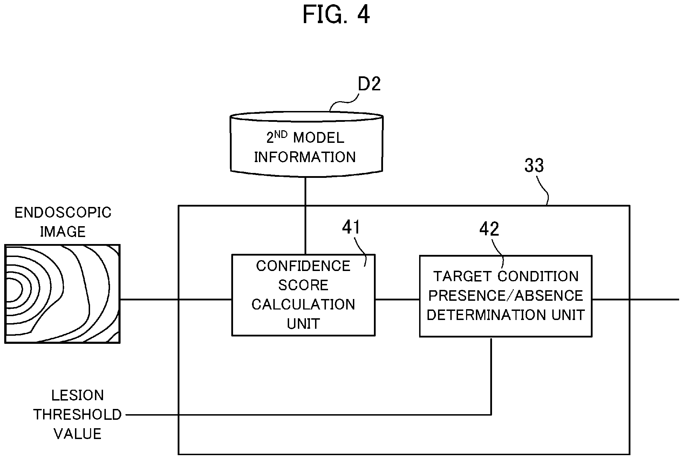

FIG. 4 shows an example of a functional block of the lesion detection unit 33. The condition detection unit 33 functionally includes a confidence score calculation unit 41, and a target condition presence / absence determination unit 42.

The confidence score calculation unit 41 inputs the endoscopic image Ia to the second model configured by referring to the second model information D2. Then, the confidence score calculation unit 41 supplies the lesion confidence score S outputted by the second model based on the inputted endoscopic image Ia to the target condition presence / absence determination unit 42. In the case where the second model is prepared for each type of the target condition, the lesion confidence score S is calculated for each type of the target condition.

On the basis of the lesion confidence score S supplied from the confidence score calculation unit 41 and the lesion threshold value supplied from the threshold value setting unit 32, the target condition presence / absence determination unit 42 determines whether or not there is a lesion part subjected to the target condition in the endoscopic image Ia. Specifically, if the lesion confidence score S is equal to or larger than the lesion threshold value, the target condition presence / absence determination unit 42 determines that there is a lesion part subjected to the target condition in the endoscopic image Ia. In contrast, if the lesion confidence score S is smaller than the lesion threshold value, the target condition presence / absence determination unit 42 determines that there is no lesion part subjected to the target condition in the endoscopic image Ia. If the lesion confidence score S is calculated for each type of the target condition, the target condition presence / absence determination unit 42 determines, for each type of the target condition, whether or not there is a lesion part. Then, the target condition presence / absence determination unit 42 outputs the lesion detection information based on the determination result to the display control unit 34. In some embodiments, the lesion detection information may include not only information on the presence or absence of the lesion part subjected to the target condition but also the lesion confidence score S and the lesion region information outputted by the second model.

The functional configuration as shown in FIGS. 3 and 4 enables the image processing device 1 to accurately detect the target condition in consideration of the background condition of the target examination. It is noted that the degree of difficulty in detecting the target condition varies depending on the background condition. For example, since the color of the surface of a large bowel having a history of an inflammatory condition is different in the color from a standard large bowel, the degree of difficulty in detecting the target condition, such as a tumor, of such a large bowel increases. Therefore, in this case, it is desired that the lesion threshold value for the lesion confidence score S be a low value. On the other hand, if the lesion threshold value is set to a low value in accordance with a large bowel under an inflammatory condition which has a high degree of detection difficulty, false detection increases in the endoscopic examination for an ordinary large bowel without an inflammatory condition. Taking the above into consideration, the image processing device 1 according to the present embodiment adaptively sets a lesion threshold value in accordance with a background condition. Thus, it is possible to accurately determine the presence or absence of the target condition regardless of the state of the subject.

Each component of the endoscope image acquisition unit 30, the background condition determination unit 31, the threshold value setting unit 32, the lesion detection unit 33, and the display control unit 34 can be realized, for example, by the processor 11 which executes a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

### (4-2) Display Examples

Next, a description will be given of the display control of the display device 2 to be executed by the display control unit 34.

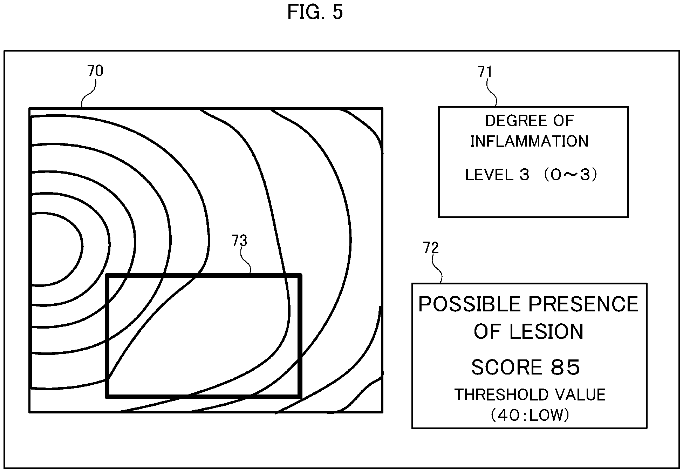

FIG. 5 shows a first display example of a display screen image displayed by the display device 2 in the endoscopic examination. The display control unit 34 of the image processing device 1 transmits the display information Ib generated on the basis of the information supplied from the other processing units 30 to 33 in the processor 11 to the display device 2, so that the display screen image shown in FIG. 5 is displayed on the display device 2.

In the first display example, the display control unit 34 of the image processing device 1 displays on the display screen image the latest endoscopic image 70 that is a moving image based on endoscopic images Ia acquired by the endoscopic image acquisition unit 30, the first display field 71 relating to the background condition, and the second display field 72 relating to the target condition.

The display control unit 34 herein displays the content based on the background condition information in the first display field 71. As an example, based on the background condition information indicating the type and degree of the detected background condition, the display control unit 34 herein displays, in the first display field 71, information to the effect that the inflammation at the level 3 on a scale of the level 0 to level 3 has occurred.

Further, based on the lesion detection information and the lesion threshold value, the display control unit 34 displays, in the second display field 72, the detection result of the lesion part subjected to the target condition, the lesion confidence score S, and the lesion threshold value. Here, on the basis of the lesion detection information indicating the presence of the lesion part subjected to the target condition and region of the lesion part subjected to the target condition, the display control unit 34 displays indication of the possible presence of a lesion part in the second display field 72 while displaying the frame 73 surrounding the region of the lesion part on the latest endoscope image 70. In this example, since the inflammation at the level 3 on a scale of levels 1 to 3 has occurred (i.e., relatively severe inflammation has occurred), the lesion threshold value is set to a low value (e.g., level 1 on a scale of levels 1 to 3). Therefore, the display control unit 34 displays on the second display field 72 information to the effect that a relatively low lesion threshold value is set. In some embodiments, if information (e.g., disease name) indicating the type of the detected target condition is included in the lesion detection information, the display control unit 34 may display information indicating the type of the target condition in the second display field 72.

In some embodiments, the display control unit 34 may display the type and degree of the background condition, the lesion threshold value, the lesion confidence score S or the like in a visual way using color coding or gauge or the like. In another example, upon determining that there is a lesion part subjected to the target condition, the display control unit 34 may highlight the latest endoscopic image 70 or/and the second display field 72 by edging effect or the like or may cause the audio output unit 16 to output warning sound or voice guidance or the like for notifying the user that a lesion part has been detected.

According to the first display example, the display control unit 34 can suitably notify the examiner of the detection result and the related information of the lesion part subjected to the target condition. Further, by displaying information regarding the lesion threshold and/or information regarding the background condition, the display control unit 34 can suitably let the examiner grasp whether or not now is in a difficult situation to detect a lesion.

### (4-3) Processing Flow

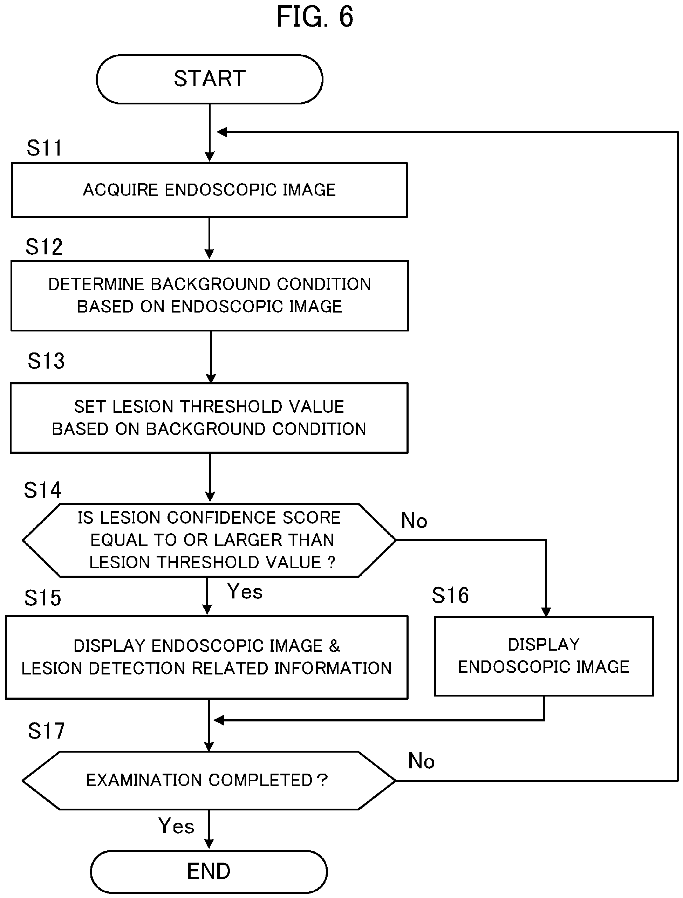

FIG. 6 is an example of a flowchart illustrating an outline of a process that is executed by the image processing device 1 during the endoscopic examination in the first example embodiment.

First, the image processing device 1 acquires the endoscopic image Ia (step S11). In this instance, the endoscopic image acquisition unit 30 of the image processing device 1 receives the endoscopic image Ia from the endoscope 3 through the interface 13.

Next, the image processing device 1 determines the background condition based on the endoscopic image Ia acquired at step S11 (step S12). In this case, the image processing device 1 determines the background condition based on the information outputted by the first model when the endoscopic image Ia is inputted into the first model, which is configured based on the first model information D1.

Then, the image processing device 1 sets the lesion threshold value based on the background condition determined at step S12 (step S13). Before or after step S13, the image processing device 1 acquires the lesion confidence score S outputted by the second model when the endoscopic image Ia is inputted into the second model, which is configured on the basis of the second model information D2.

Then, the image processing device 1 determines whether or not the lesion confidence score S is equal to or larger than the lesion threshold value (step S14). Then, upon determining that the lesion confidence score S is equal to or greater than the lesion threshold value (step S14; Yes), the image processing device 1 determines that there is a lesion part subjected to the target condition, and displays the endoscopic image Ia acquired at step S11 and the information related to the detected lesion on the display device 2 (step S15). In this case, the image processing device 1 may display, as information related to the detected lesion, for example, information to the effect that a lesion part is detected, the region of the detected lesion part, the lesion confidence score S, and/or the established lesion threshold value.

On the other hand, upon determining that the lesion confidence score S is less than the lesion threshold value (step S14; No), the image processing device 1 determines that there is no lesion part subjected to the target condition and displays the endoscopic image Ia acquired at step S11 on the display device 2 (step S16). Also in this instance, the image processing device 1 may display the calculated lesion confidence score S, the set lesion threshold, and the like in the same manner as in the process at step S15.

Then, the image processing device 1 determines whether or not the endoscopic examination has been completed, after the process at step S15 or step S16 (step S17). For example, upon detecting a predetermined input or the like to the input unit 14 or the operation unit 36, the image processing device 1 determines that the endoscopic examination has been completed. Upon determining that the endoscopic examination has been completed (step S17; Yes), the image processing device 1 ends the process of the flowchart. On the other hand, upon determining that the endoscopic examination has not been completed (step S17; No), the image processing device 1 proceeds back to the process at step S11. Then, the image processing device 1 proceeds with the processes at step S11 to step S16 using the endoscopic image Ia newly generated by the endoscope 3.

### (5) Modifications

Next, modifications suitable for the above-described embodiment will be described. The following modifications may be applied to the embodiments described above in combination.

### (First Modification)

Instead of or in addition to setting the lesion threshold value based on the background condition, the image processing device 1 may set the lesion threshold value based on a part (also referred to as "photographed part") currently photographed by the endoscope 3.

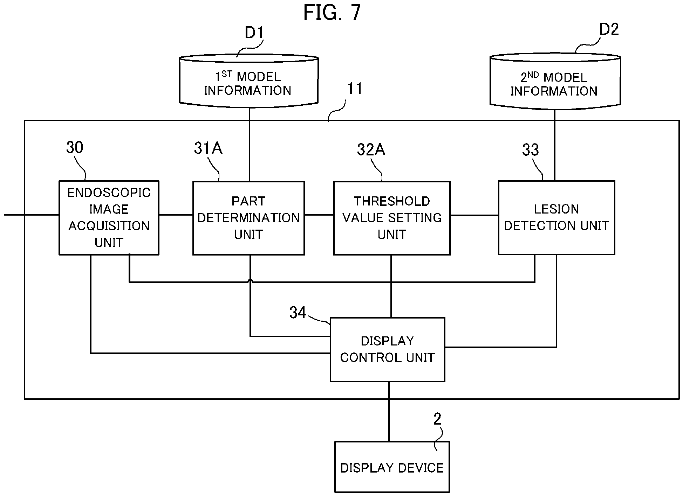

FIG. 7 is an example of functional blocks of the processor 11 of the image processing device 1 according to the first modification. The processor 11 according to the first modification functionally includes an endoscopic image acquisition unit 30, a part determination unit 31A, a threshold value setting unit 32A, a lesion detection unit 33, and a display control unit 34. The endoscope image acquisition unit 30, the lesion detection unit 33, and the display control unit 34 are identical to the endoscope image acquisition unit 30, the lesion detection unit 33, and the display control unit 34, which are shown in FIG. 3, and therefore the detailed description thereof will be omitted.

Based on the endoscopic image Ia supplied from the endoscopic image acquisition unit 30, the part determination unit 31A determines a photographed part of the examination target serving as a subject in the endoscopic image Ia. For example, when the examination target is the large bowel, the portion determination unit 31A classifies the photographed part into any of the following parts: a rect (a lower rect, an upper rect, and a rectal sigmoid portion), a sigmoid colon, a descending colon, a transverse colum, an ascending colon, a caecum, a cyclone, an ecliptic valve, and an appendix. The classes for the classification need not be divided according to the name, and the classes may be flexibly set according to the difficulty in detecting the lesion.

For example, on the basis of the first model, which is configured by referring to the first model information D1, and the endoscopic image Ia, the part determination unit 31A may determine the photographed part. In this case, the first model is, for example, a classification model configured to output a classification result regarding a photographed part indicated by the inputted endoscopic image when an endoscopic image is inputted to the model, and the first model information D1 includes parameters required for configuring the first model.

In some embodiments, the part determination unit 31A receives information regarding the insertion state of the endoscope 3 (e.g., the length of the shaft 37 inserted into the examination target) from the endoscope 3 or a sensor thereof (not shown) or the input unit 14 and determines the photographed part in accordance with the insertion state. In this case, for example, in the memory 12 or the like, information indicating the relation between the insertion state of the endoscope 3 and the photographed part is stored in advance.

The threshold value setting unit 32A sets a lesion threshold value based on the determined photographed part. In this case, for example, relation information (e.g., a look-up table or expression) indicating a relation between a photographed part and a lesion threshold value to be set is stored in the memory 12, and the threshold value setting unit 32 sets the lesion threshold value corresponding to the determined photographed part with reference to the relation information. In this case, the above-described relation information indicates that the lesion threshold value increases (becomes strict) with increasing degree of difficulty in detecting a lesion in the photographed part.

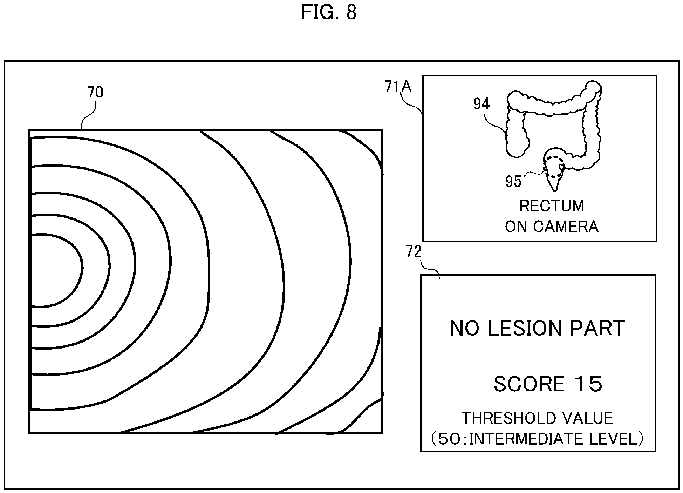

FIG. 8 shows a second display example of a display screen image displayed on the display device 2 in the endoscopic examination according to the first modification. The display control unit 34 of the image processing device 1 transmits the display information Ib generated on the basis of the information supplied from the other processing units in the processor 11 to the display device 2, so that the display screen image according to the second display example is displayed on the display device 2.

In the second display example, the display control unit 34 of the image processing device 1 displays the latest endoscopic image 70, the first display field 71A relating to the photographed part, and the second display field 72 relating to the target condition on the display screen image. In the latest endoscopic image 70, similarly to the first display example, a moving image based on the latest endoscopic image Ia acquired by the endoscopic image acquisition unit 30 is displayed.

The display control unit 34 herein displays information on the photographed part determined by the part determination unit 31A on the first display field 71A. Here, as an example, the display control unit 34 displays a three-dimensional model 94 representing the whole large bowel that is the target organ of examination, and displays the name (rectum, herein) and the position (see the broken line frame 95) of the photographed part on the first display field 71A in association with the three dimensional model 94. In this instance, the display control unit 34 performs three-dimensional reconstruction of the examination target by a SfM or the like using a time-series endoscopic images and identifies the photographed part through image registration (matching) between the reconstruction and the standard model of the examination target stored in advance in the memory 12. Thus, the display control unit 34 displays a model representing the entire examination target in which the photographed part is clearly indicated, which enables the examiner to suitably grasp the position of the photographed part.

Further, based on the lesion detection information and the lesion threshold value, the display control unit 34 displays, in the second display field 72, information indicating that there is no lesion part subjected to the target condition, the lesion confidence score S, and the lesion threshold value.

Thus, the image processing device 1 according to the first modification can set a lesion threshold value in consideration of the difficulty in detecting the lesion part according to the photographed part and accurately perform the detection of the lesion part.

In some embodiments, instead of or in addition to setting the lesion threshold value based on the background condition or/and the photographed part, the image processing device 1 may set the lesion threshold value based on the presence or absence of a residue or the color of the examination target. For example, in a case of setting the lesion threshold value based on the presence or absence of the residue, the image processing device 1 sets the lesion threshold value in accordance with the amount of the residue. For example, the image processing device 1 may decrease the lesion threshold value with an increase in the amount of residue. In some embodiments, based on the endoscopic image Ia and the learning model trained in advance, the image processing device 1 may determine whether or not there is a residue and the amount of residue when the residue has been detected. In this case, for example, the memory 12 stores, in advance, the parameters of the model trained to output the presence or absence of a residue (and the amount of the residue when a residue has been detected) in the examination target indicated by the inputted endoscope image Ia when an endoscope image Ia is inputted to the model. Similarly, in a case of setting the lesion threshold value based on the color of the examination target, the image processing device 1 sets the lesion threshold value, for example, in accordance with the RGB ratio of the endoscopic image Ia. In this instance, the memory 12 stores, in advance, information indicating the relation between the RGB ratio and the lesion threshold value to be set.

Thus, the image processing device 1 can accurately perform detection of the lesion part even when the lesion threshold value is set on the basis of the state (residue, color, and the like) of the examination target other than the background condition.

### (Second Modification)

The image processing device 1 may detect the lesion subjected to the target condition based on the state (including background condition, residue, and/or color) or the photographed part, without setting the lesion threshold value.

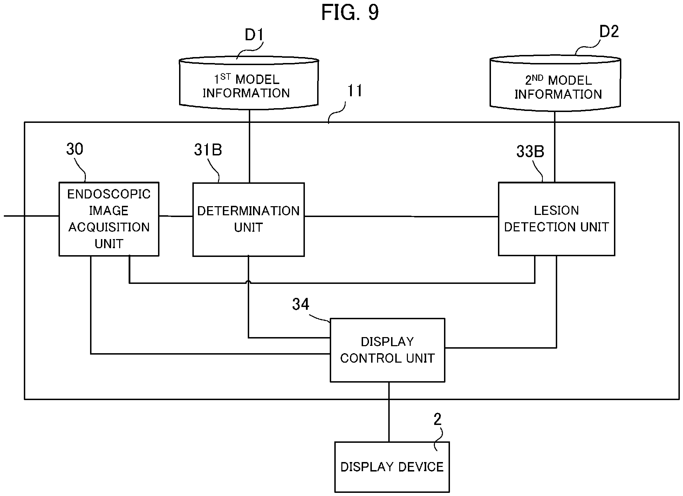

FIG. 9 is an example of a functional block diagram of the processor 11 according to the first example of the second modification. The processor 11 according to the first example of the second modification functionally includes an endoscopic image acquisition unit 30, a determination unit 31B, a lesion detection unit 33B, and a display control unit 34. Since the endoscope image acquisition unit 30 and the display control unit 34 are identical to the endoscope image acquisition unit 30 and the display control unit 34 shown in FIG. 3, the detailed description thereof will be omitted.

The determination unit 31B corresponds to the background condition determination unit 31 in FIG. 3 or the part determination unit 31A according to the first modification, and determines the state (including the background condition, the residue, and/or the color) of the examination target or the photographed part of the examination target. Then, the determination unit 31B supplies the determination result to the lesion detection unit 33B.

The lesion detection unit 33B detects the lesion part subjected to the target condition, based on the determination result from the determination unit 31B, the endoscopic image Ia supplied from the endoscopic image acquisition unit 30, and the second model configured by the second model information D2. In this case, for example, the lesion detection unit 33B converts the determination result from the determination unit 31B and the endoscopic image Ia into data in a predetermined tensor format, and detects the lesion part subjected to the target condition, based on the information outputted by the second model when the data after the conversion is inputted into the second model. In this case, for example, the second model is preliminarily trained to output, when data in the predetermined tensor format is inputted thereto, at least the lesion confidence score S as to the presence of the lesion part subjected to the target condition. The second model information D2 including the learned parameters is stored in advance in the memory 12. The data in the predetermined tensor format may be, for example, data in which data indicating the determination result from the determination unit 31B is superimposed on the endoscopic image Ia in the channel direction. The second model in the first example of the second modification corresponds to a model which learned a relation between input data and the presence or absence of a lesion part in an endoscopic image included in the input data.

Thus, according to the first example of the second modification, the image processing device 1 includes information regarding the state of the examination target, such as the background condition, and/or the photographed part of the examination target in the input data of the second model. This also allows the image processing device 1 to perform accurate lesion detection in consideration of the state of the examination target, such as a background condition, and the photographed part of the examination target.

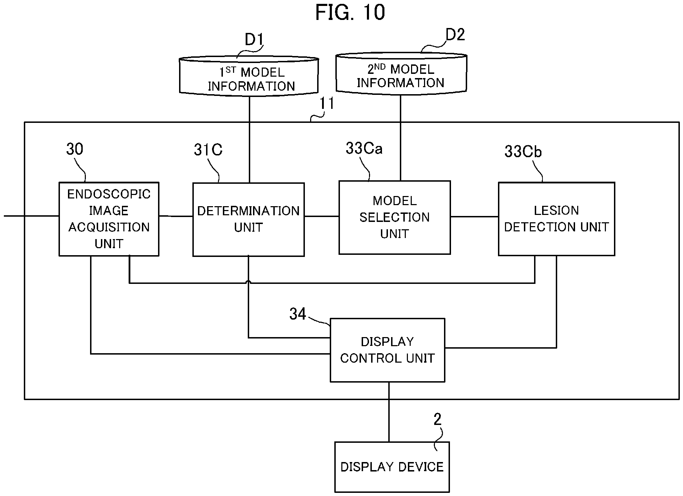

FIG. 10 is a functional block diagram of the processor 11 according to the second example of the second modification. The processor 11 according to the second example of the second modification functionally includes an endoscopic image acquisition unit 30, a determination unit 31C, a model selection unit 33., a lesion detection unit 33Cb, and a display control unit 34. Since the endoscope image acquisition unit 30 and the display control unit 34 herein are identical to the endoscope image acquisition unit 30 and the display control unit 34 shown in FIG. 3, the detailed description thereof will be omitted.

The determination unit 31C corresponds to the determination unit 31B according to the first example of the second modification, and determines the state of the examination target (including background condition, residue, and/or color) or the photographed part of the examination target. The determination unit 31C supplies the determination result to the model selection unit 33..

The model selection unit 33. selects, based on the determination result from the determination unit 31C, the second model to be used by the lesion detection unit 33Cb. Then, the model selection unit 33. extracts the parameters of the selected second model from the second model information D2, and supplies the parameters to the lesion detection unit 33Cb. In this instance, parameters of a plurality of candidates (also referred to as "second model candidates") for the second model which are possibly used by the lesion detection unit 33Cb are stored in the second model information D2. The second model candidates are linked with classes classified according to the state of the examination target or the photographed part determined by the determination unit 31C, respectively. Then, the second model candidates are trained in advance based on the training data including endoscopic images according to the linked classes and the correct answer data indicating the presence or absence of a lesion in each of the endoscopic images. Then, the model selection unit 33. extracts the parameters of the second model candidate linked with the class corresponding to the determination result generated by the determination unit 31C from the second model information D2, and supplies the extracted parameters to the lesion detection unit 33Cb as the parameters of the second model to be used.

The lesion detection unit 33Cb inputs the endoscopic image Ia to the second model configured based on the parameters supplied from the model selection unit 33.. Then, the lesion detection unit 33Cb detects the presence or absence of the lesion part based on the information outputted by the second model in response to the input of the endoscopic image Ia, and supplies the lesion detection information indicating the detection result to the display control unit 34.

Thus, in the second example of the second modification, the image processing device 1 switches the second model to be used to perform the lesion detection according to the state of the examination target such as the background condition or the photographed part. This also allows the image processing device 1 to perform accurate lesion detection in consideration of the state of the examination target such as the background condition and the photographed part thereof.

### (Third Modification)

After the examination, the image processing device 1 may process the moving image data configured by the endoscopic images Ia generated during the endoscopic examination.

For example, the image processing device 1 sequentially performs the process according to the flowchart in FIG. 6 with respect to each endoscopic image Ia in the time series constituting the moving image data when the moving image data to be processed is specified based on the user input or the like through the input unit 14 at an arbitrary timing after the examination. Upon determining at step S17 that the process of the target moving image data has ended, the image processing device 1 terminates the processing of the flowchart. In contrast, upon determining that the process of the target moving image data has not ended, the image processing device 1 returns the process to step S11 and performs process according to the flowchart for the subsequently captured endoscopic image Ia.

### (Fourth Modification)

The first model information D1 and the second model information D2 may be stored in a storage device separate from the image processing device 1.

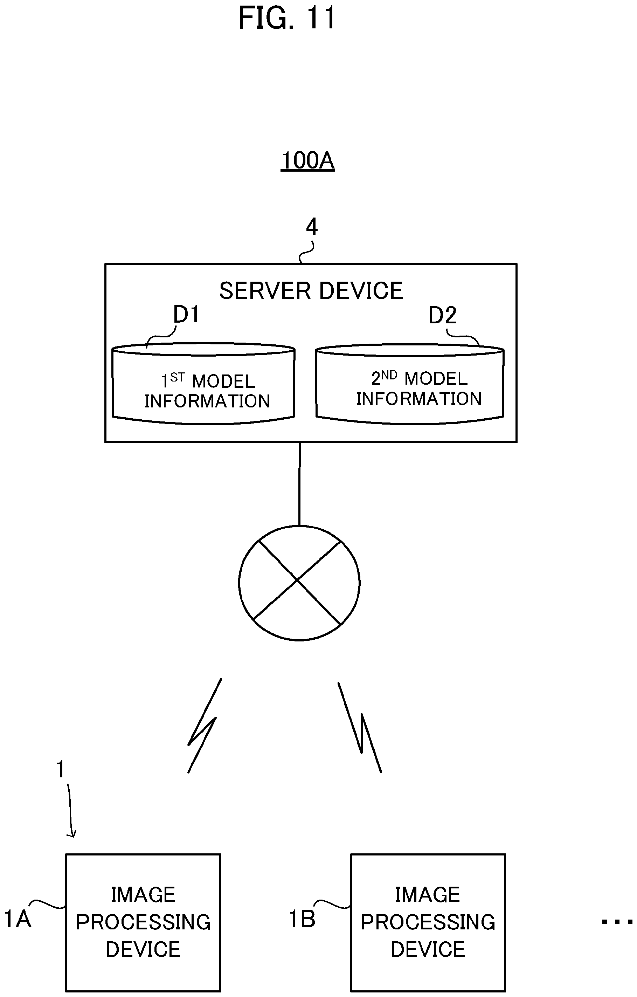

FIG. 11 is a schematic configuration diagram illustrating the endoscopic examination system 100A according to the fourth modification. For simplicity, the display device 2 and the endoscope 3 and the like are not shown. The endoscopic examination 100A includes a server device 4 that stores the first model information D1 and the second model information D2. Further, the endoscopic examination system 100A includes a plurality of image processing devices 1 (1A, 1B, ...) capable of data communication with the server device 4 via a network.

In this instance, the image-processing devices 1 refer to the first model information D1 and the second model information D2 through the network. In this case, the interface 13 of each image processing device 1 includes a communication interface such as a network adapter for data communication. In this configuration, each image processing device 1 can suitably execute the processing related to the lesion detection by referring to the first model information D1 and the second model information D2 in the same way as in the above-described example embodiment.

### (Fifth Modification)

The target to be detected by the second model in the endoscopic examination system 100 is not limited to a lesion part, but may be any part (point) of interest which needs to be noticed by the examiner. Examples of such a part of interest include a lesion part, an inflammation part, a part with an operating mark or other cuts, a part with a fold or a protrusion, a part on the wall surface of the lumen where the tip unit 38 of the endoscope 3 tends to get contact (caught).

### <Second Example Embodiment>

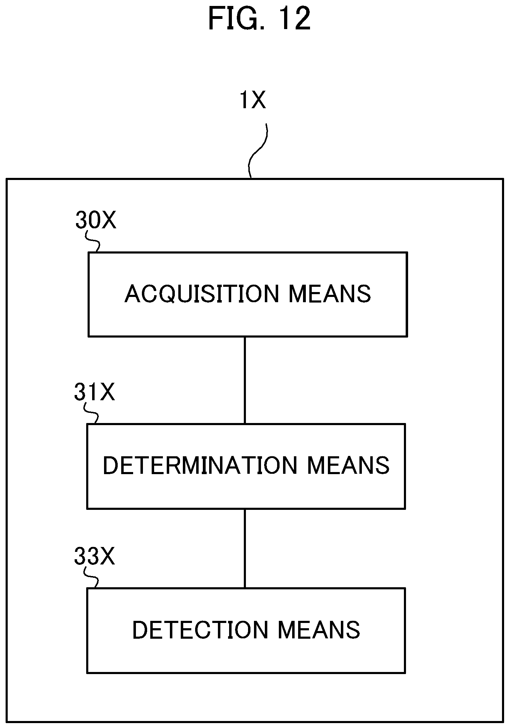

FIG. 12 is a block diagram of an image processing device 1X according to the second example embodiment. The image processing device 1X includes an acquisition means 30X, a determination means 31X, and a detection means 33X. The image processing device 1X may be configured by a plurality of devices.

The acquisition means 30X is configured to acquire an endoscopic image obtained by photographing an examination target by a photographing unit provided in an endoscope. Examples of the acquisition means 30X include the endoscopic image acquisition unit 30 in the first example embodiment (including modifications, hereinafter the same). The acquisition means 30X may be configured to immediately acquire the endoscopic image generated by the photographing unit, or may be configured to acquire, at a predetermined timing, the endoscopic image stored in the storage device generated by the photographing unit in advance.

The determination means 31X is configured to determine, based on the endoscopic image, a state of the examination target or a photographed part of the examination target. Examples of the determination means 31X include the background condition determination unit 31, the part determination unit 31A, and the determination units 31B and 31C in the first example embodiment.

The detection means 33X is configured to detect, based on the state or the photographed part and the endoscopic image, a part of interest to be noticed in the examination target. The term "detection" herein only requires to make a determination as to presence or absence of a part of interest in the examination target and does not necessarily require to identify the region of the part of interest. Examples of the detection means 33X include the lesion detection unit 33, the lesion detection unit 33B, and the lesion detection unit 33Cb in the first example embodiment.

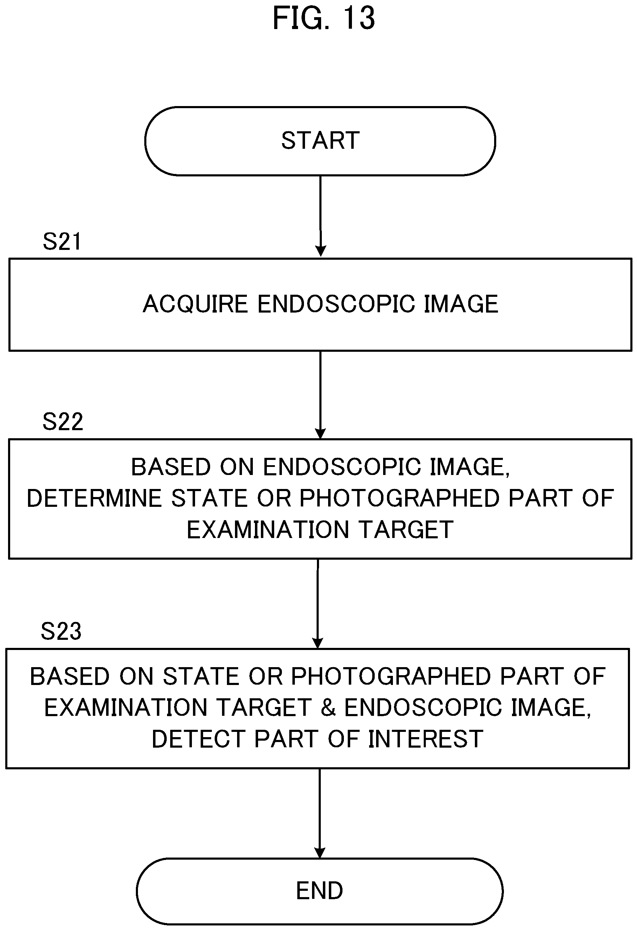

FIG. 13 is an example of a flowchart showing a processing procedure in the second example embodiment. The acquisition means 30X acquires an endoscopic image obtained by photographing an examination target by a photographing unit provided in an endoscope (step S21). Next, the determination means 31X determines, based on the endoscopic image, a state of the examination target or a photographed part of the examination target (step S22). Then, the detection means 33X detects, based on the state or the photographed part and the endoscopic image, a part of interest to be noticed in the examination target (step S23).

According to the second example embodiment, the image processing device 1X can accurately detect a region of interest from an endoscopic image obtained by photographing the examination target.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

The whole or a part of the example embodiments described above (including modifications, the same applies hereinafter) can be described as, but not limited to, the following Supplementary Notes.

### [Supplementary Note 1]

An image processing device comprising:
an acquisition means configured to acquire an endoscopic image obtained by photographing an examination target by a photographing unit provided in an endoscope;
a determination means configured to determine, based on the endoscopic image, a state of the examination target or a photographed part of the examination target; and
a detection means configured to detect, based on the state or the photographed part and the endoscopic image, a part of interest to be noticed in the examination target.

### [Supplementary Note 2]

The image processing device according to Supplementary Note 1,
wherein the determination means is configured to determine, as the state of the examination target, at least, a background condition, which is a condition of the examination target other than a target condition to be detected as the part of interest, and
wherein the detection means is configured to detect the target condition based on the background condition and the endoscopic image.

### [Supplementary Note 3]

The image processing device according to Supplementary Note 1 or 2,
wherein the determination means is configured to determine, as the state of the examination target, at least, a presence or absence of a residue in the target examination, and
wherein the detection means is configured to detect the part of interest based on the presence or absence of the residue and the endoscope image.

### [Supplementary Note 4]

The image processing device according to any one of Supplementary Notes 1 to 3,
wherein the determination means is configured to determine, as the state of the examination target, at least, a color of the examination target, and
wherein the detection means is configured to detect, based on the color and the endoscopic image, the part of interest.

### [Supplementary Note 5]

The image processing device according to any one of Supplementary Notes 1 to 4, further comprising
a threshold value setting means configured to set, based on the state or the photographed part, a threshold value for comparing with a score,
the score indicating a confidence level as the part of interest,
wherein the detection means is configured to detect the part of interest, based on the score calculated based on the endoscopic image and the threshold value.

### [Supplementary Note 6]

The image processing device according to Supplementary Note 5, further comprising
a display control means configured to cause a display device to display at least one of
information regarding the threshold value and/or
information regarding the state or the photographed part.

### [Supplementary Note 7]

The image processing device according to Supplementary Note 6,
wherein the display control means is configured to cause the display device to display, as the information regarding the photographed part, a model representing a whole of the examination target with a clear indication of the photographed part.

### [Supplementary Note 8]

The image processing device according to any one of Supplementary Notes 1 to 7,
wherein the detection means is configured to detect the part of interest, based on
   input data based on the state or the photographed part and the endoscopic image and
   a model to which the input data is inputted, and
wherein the model is a model learned a relation between
   the input data and
   a presence or absence of the part of interest in the endoscopic image included in the input data.

### [Supplementary Note 9]

The image processing device according to any one of Supplementary Notes 1 to 7,
wherein the detection means is configured to detect the part of interest, based on
   a model selected based on the state or the photographed part and
   the endoscopic image,
wherein the model is a model which learned a relation between
   an endoscopic image inputted to the model and
   a presence or absence of the part of interest in the endoscopic image.

### [Supplementary Note 10]

An image processing method executed by a computer, comprising:
acquiring an endoscopic image obtained by photographing an examination target by a photographing unit provided in an endoscope;
determining, based on the endoscopic image, a state of the examination target or a photographed part of the examination target; and
detecting, based on the state or the photographed part and the endoscopic image, a part of interest to be noticed in the examination target.

### [Supplementary Note 11]

A storage medium storing a program executed by a computer, the program causing the computer to:
acquire an endoscopic image obtained by photographing an examination target by a photographing unit provided in an endoscope;
determine, based on the endoscopic image, a state of the examination target or a photographed part of the examination target; and
detect, based on the state or the photographed part and the endoscopic image, a part of interest to be noticed in the examination target.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1A, 1B, 1X: Image processing device
- 2: Display device
- 3: Endoscope
- 11: Processor
- 12: Memory
- 13: Interface
- 14: Input unit
- 15: Light source unit
- 16: Audio output unit
- 100, 100A: Endoscopic examination system

## Claims

1. An image processing device comprising:
an acquisition means configured to acquire an endoscopic image obtained by photographing an examination target by a photographing unit provided in an endoscope;
a determination means configured to determine, based on the endoscopic image, a state of the examination target or a photographed part of the examination target; and
a detection means configured to detect, based on the state or the photographed part and the endoscopic image, a part of interest to be noticed in the examination target.

2. The image processing device according to claim 1,
wherein the determination means is configured to determine, as the state of the examination target, at least, a background condition, which is a condition of the examination target other than a target condition to be detected as the part of interest, and
wherein the detection means is configured to detect the target condition based on the background condition and the endoscopic image.

3. The image processing device according to claim 1 or 2,
wherein the determination means is configured to determine, as the state of the examination target, at least, a presence or absence of a residue in the target examination, and
wherein the detection means is configured to detect the part of interest based on the presence or absence of the residue and the endoscope image.

4. The image processing device according to any one of claims 1 to 3,
wherein the determination means is configured to determine, as the state of the examination target, at least, a color of the examination target, and
wherein the detection means is configured to detect, based on the color and the endoscopic image, the part of interest.

5. The image processing device according to any one of claims 1 to 4, further comprising
a threshold value setting means configured to set, based on the state or the photographed part, a threshold value for comparing with a score,
the score indicating a confidence level as the part of interest,
wherein the detection means is configured to detect the part of interest, based on the score calculated based on the endoscopic image and the threshold value.

6. The image processing device according to claim 5, further comprising
a display control means configured to cause a display device to display at least one of
information regarding the threshold value and/or
information regarding the state or the photographed part.

7. The image processing device according to claim 6,
wherein the display control means is configured to cause the display device to display, as the information regarding the photographed part, a model representing a whole of the examination target with a clear indication of the photographed part.

8. The image processing device according to any one of claims 1 to 7,
wherein the detection means is configured to detect the part of interest, based on
input data based on the state or the photographed part and the endoscopic image and
a model to which the input data is inputted, and
wherein the model is a model learned a relation between
the input data and
a presence or absence of the part of interest in the endoscopic image included in the input data.

9. The image processing device according to any one of claims 1 to 7,
wherein the detection means is configured to detect the part of interest, based on
a model selected based on the state or the photographed part and
the endoscopic image,
wherein the model is a model which learned a relation between
an endoscopic image inputted to the model and
a presence or absence of the part of interest in the endoscopic image.

10. An image processing method executed by a computer, comprising:
acquiring an endoscopic image obtained by photographing an examination target by a photographing unit provided in an endoscope;
determining, based on the endoscopic image, a state of the examination target or a photographed part of the examination target; and
detecting, based on the state or the photographed part and the endoscopic image, a part of interest to be noticed in the examination target.

11. A storage medium storing a program executed by a computer, the program causing the computer to:
acquire an endoscopic image obtained by photographing an examination target by a photographing unit provided in an endoscope;
determine, based on the endoscopic image, a state of the examination target or a photographed part of the examination target; and
detect, based on the state or the photographed part and the endoscopic image, a part of interest to be noticed in the examination target.
